# EUROPEAN PATENT SPECIFICATION

(11) **EP 3 720 465 B1**
(45) Date of publication and mention of the grant of the patent: **23.07.2025**
(21) Application number: 18826475.8
(22) Date of filing: 07.12.2018
(51) Int. Cl.: A61K 38/13, A61K 47/34, A61K 9/08, A61K 31/542, A61P 27/02

(54) **HYPOTONIC HYDROGEL FORMULATIONS FOR ENHANCED TRANSPORT OF ACTIVE AGENTS AT MUCOSAL SURFACES**
HYPOTONISCHE HYDROGELFORMULIERUNGEN ZUM VERBESSERTEN WIRKSTOFFTRANSPORT AN MUKOSALE FLÄCHEN
FORMULATION EN FORME DE HYDROGÈLE HYPOTONIQUE POUR LE TRANSPORT AMELIORÉ AUX SURFACES MUCOSALES

(30) Priority: 08.12.2017 US 201762596578 P; 07.02.2018 US 201862627559 P
(43) Date of publication of application: 14.10.2020
(73) Proprietor: The Johns Hopkins University, Baltimore, MD 21218 (US)
(72) Inventor: ENSIGN, Laura, Towson Maryland 21286 (US); HANES, Justin, Baltimore Maryland 21212 (US); DATE, Abhijit, Baltimore Maryland 21215 (US); KIM, Yoo Chun, Baltimore Maryland 21205 (US)
(74) Representative: Potter Clarkson
(86) International application number: PCT/US2018/064441
(87) International publication number: WO 2019/113425

(56) References cited:
- WO-A1-2015/127368
- WO-A1-2016/123125
- US-A1- 2014 107 173

## Description

### CROSS-REFERENCED TO RELATED APPLICATIONS

This application claims the benefit of and priority to U.S. Provisional Application No. 62/596,578 filed December 8, 2017 and U.S. Provisional Application No. 62/627,559 filed February 7, 2018.

### STATEMENT REGARDING FEDERALLY SPONSORED

### RESEARCH OR DEVELOPMENT

This invention was made with government support under Grant No. RO1DK107806 awarded by the National Institutes of Health. The government has certain rights in the invention.

### FIELD OF THE INVENTION

This invention is generally in the field of formulations for enhanced drug delivery, in particular drug delivery at mucosal surfaces.

### BACKGROUND OF THE INVENTION

The mucosa is a membrane that lines various cavities in the body, *e.g.,* mouth, gut, uterus, vagina, colon, anal canal, trachea, lungs, bladder, etc. As used herein, skin may be considered a mucosal surface. The mucosa consists of the *epithelium* itself and also the supporting loose connective tissue, called *lamina propria,* immediately beneath the epithelium. Deeper connective tissue which supports the mucosa is called the *submucosa.* In the GI tract, there is a thin layer of smooth muscle, the *muscularis mucosae,* at the boundary between mucosa and submucosa.

The mucosal surfaces of the body are selectively permeable. Mucosal barrier injuries, such as oral and gastrointestinal mucositis, are a common complication following cytoreductive cancer therapy and radiotherapy (Sonis et al., Cancer Supplement, 100(9):1995-2023, 2004). Some capsid viruses can diffuse through mucus as rapidly as through water and thereby penetrate to the epithelium even though they have to diffuse 'upstream' through mucus that is being continuously secreted. These viruses are smaller than the mucus mesh spacing, and have surfaces that do not stick to mucus (Cone R. A., Adv. Drug Deliv Rev, 61(2):75-85, 2009). For example, women are disproportionately infected with HIV, partly owing to a lack of female-controlled prevention methods (Ndesendo et al., AAPS PharmSciTech, 9:505-520, 2008). An easily administered, discreet, and effective method for protecting women against vaginal HIV transmission could prevent millions of infections worldwide. However, vaginal folds, or "rugae", that accommodate expansion during intercourse and childbirth, are typically collapsed by intra-abdominal pressure, making the surfaces of these folds less accessible to drugs and drug carriers (Alexander et al., Sex Transm Dis, 29:655-664, 2004). Poor distribution into the vaginal folds, even after simulated intercourse, has been cited as a critical factor for failure to protect susceptible vaginal surfaces from infection. Distribution over the entire susceptible target surface has been proven to be important for preventing and treating infections. Additionally, to increase user acceptability, drug delivered to the vagina should be retained in the vaginal tract at effective concentrations over extended periods of time.

Achieving sustained local drug concentrations is challenging because the vaginal epithelium is highly permeable to small molecules and also because soluble drug dosage forms (gels, creams) can be expelled by intra-abdominal pressure and ambulation. Lastly, drug delivery methods must be safe and non-toxic to the vaginal epithelium. Improvements in the distribution, retention, and safety profile of vaginal dosage forms may lead to a substantial increase in efficacy and decrease in the side effects caused by largely ineffective systemic treatments for cervicovaginal infections and diseases (Thigpen T. Cancer J. 9:245-432, 2003; Robinson et al., Obstet Gynecol, 99:777-784, 2002).

Sustained drug delivery to the mucosal surfaces of the body has potential for improving the treatment and prevention of many diseases, including sexually transmitted infections, inflammatory bowel disease, lung inflammation, and degenerative eye conditions to name only a few. Achieving sustained prophylactic or therapeutic drug concentrations using traditional soluble dosage forms remains challenging due to degradation, rapid shedding, and rapid systemic absorption of drug. There is an urgent need for compositions for delivery to mucosal surfaces that provide a physical barrier to pathogen entry. Also, there is an unmet need for compositions for mucosal delivery that offer retention and sustained release of prophylactic, therapeutic or diagnostic agents at mucosal surfaces.

Therefore, it is an object of the invention to provide improved compositions for delivery of active agents with greater efficacy and safety to mucosal surfaces that act as barriers to pathogen transport into the mucosa.

It is a further object of the present invention to provide improved compositions for delivery to mucosal surfaces that allow retention and sustained release of prophylactic, therapeutic or diagnostic agents at mucosal surfaces.

It is still a further object of the present invention to provide methods of making the improved compositions for delivery to mucosal surfaces.

WO 2016/123125 A1 was published 4 August 2016 and describes hypotonic hydrogel formulations for enhanced transport of active agents at mucosal surfaces.

US 2014/107173 A1 was published 17 April 2014 and describes α-2 adrenergic receptor agonist compositions for treating glaucoma and other intraocular conditions.

### SUMMARY OF THE INVENTION

Hypotonic gelling vehicles are used as solubilizing agents for drugs with lower water solubility. This approach is in contrast to using a nanoparticle or nanocrystal to improve the solubility/dissolution of a hydrophobic drug. Solubilizing the drugs enhances mucosal penetration, while the hypotonic gelling vehicle further improves distribution and penetration. Many drugs have minimal water solubility, limiting their delivery to mucosal surfaces.

Solubilizing drugs in thermosensitive gelling vehicles improves distribution, retention, and penetration of drugs delivered topically when administered hypotonically. Hypotonic formulations of hydrogel forming polymers, in which the hydrogel forming polymers are (i) between greater than 12 and less than 24% Pluronic^{®} F98 in an aqueous excipient, or (ii) between 10 and 18% Pluronic^{®} F127, have been developed for enhanced delivery of therapeutic, diagnostic, prophylactic or other agents, to a mucosal or epithelial surface of the eye. The polymers are administered in a hypotonic solution at a concentration less than their normal critical gelling concentration (CGC). Typically, a Poloxamer gel administered into the vagina or colorectum at its CGC will form a "plug" of gel in the lumen. In contrast, fluid from a hypotonically-administered Poloxamer solution below the CGC, as defined in the claims, will be absorbed by the epithelial surface, drawing the Poloxamer into the mucus layers and up against the epithelium where it then becomes concentrated enough to gel, thereby enhancing and facilitating delivery of agents to the epithelial cells. As the Poloxamer is concentrated at the tissue/mucosal interface, it mixes with mucus and gels up against the epithelial surface. The endogenous mucin glycopolymers affect the gelling properties of the hypotonic gelling agents, including the concentration of gelling agent needed to gel and the pore structure of the resulting gel/mucin mixture.

Studies demonstrate the advantages of these hypotonic polymeric gel formulations for drug delivery, especially through mucosal epithelia.

### BRIEF DESCRIPTION OF THE DRAWINGS

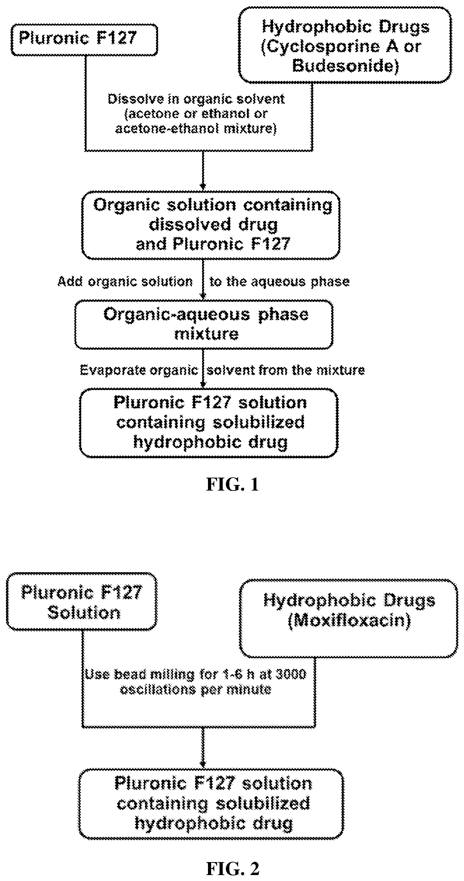

**Figure 1** is a scheme for solubilization of hydrophobic drugs (*e.g.* cyclosporine A or budesonide) by a solvent evaporation method.
**Figure 2** is a scheme for solubilization of hydrophobic drugs (*e.g.* moxifloxacin) by a bead milling method.

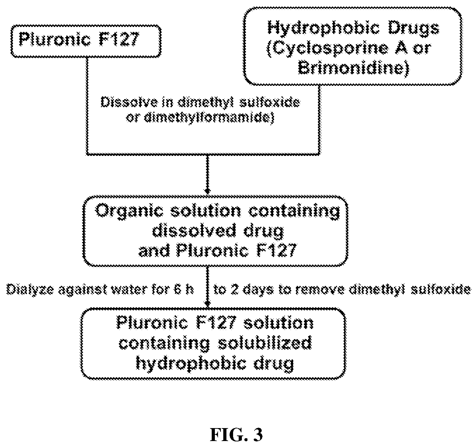

**Figure 3** is a scheme for solubilization of hydrophobic drugs (*e.g.* cyclosporine A or brimonidine) by a dialysis method.

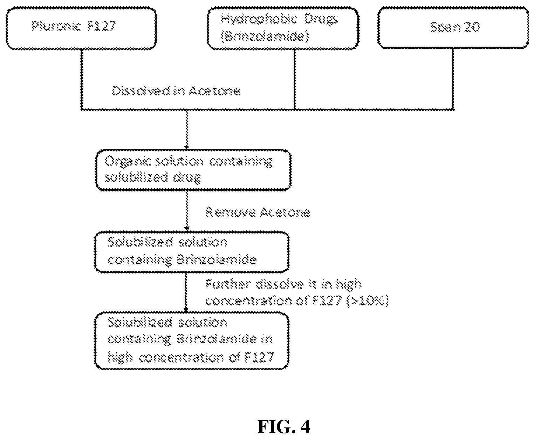

**Figure 4** is scheme for solubilization of hydrophobic drugs (*e.g.* brinzolamide) by a solvent evaporation method.

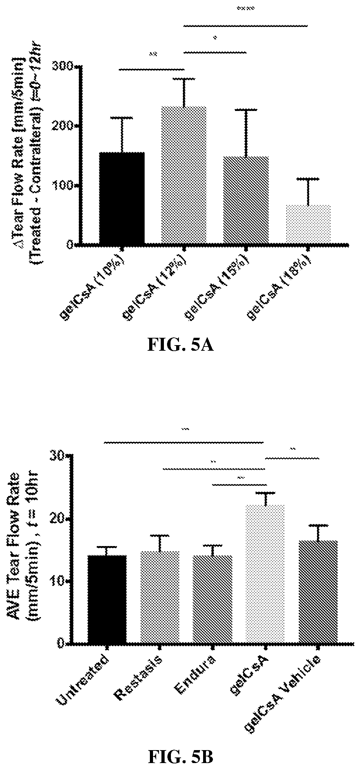

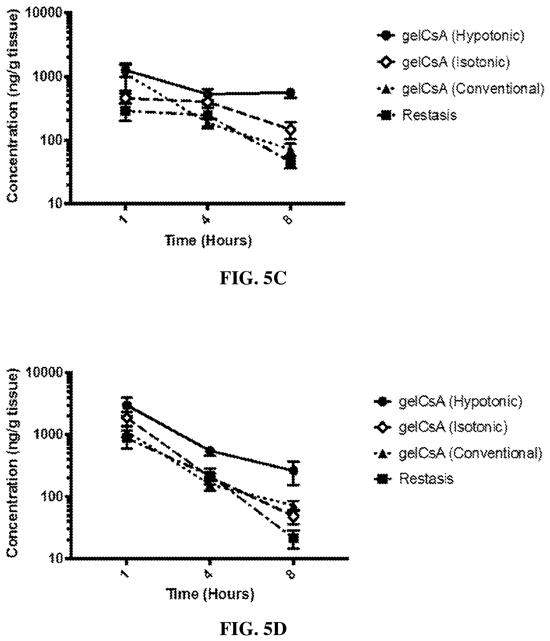

**Figure 5A** is a graph showing the effect of various concentrations of Pluronic^{®} F127 (10%, 12%, 15%, and 18%) on the cumulative increase in tear production induced by cyclosporine in healthy rabbit eyes for 12 h after a single drop. **Figure 5B** is a graph of the tear production in the healthy rabbit eye 10 h after each of 5 daily doses of gelCsA, the gel vehicle containing no drugs (gelCsA Vehicle), Restasis, the Restasis vehicle (Endura), or no treatment (Untreated). Figures 5C and 5D are line graphs showing concentrations of cyclosporine in rat cornea (**Figure 5C**) and conjunctiva (**Figure 5D**) tissue after administering cyclosporine eye drops formulated as gelCsA (Hypotonic), gelCsA (Isotonic), gelCsA (Conventional), or Restasis over the period of 8 hours.

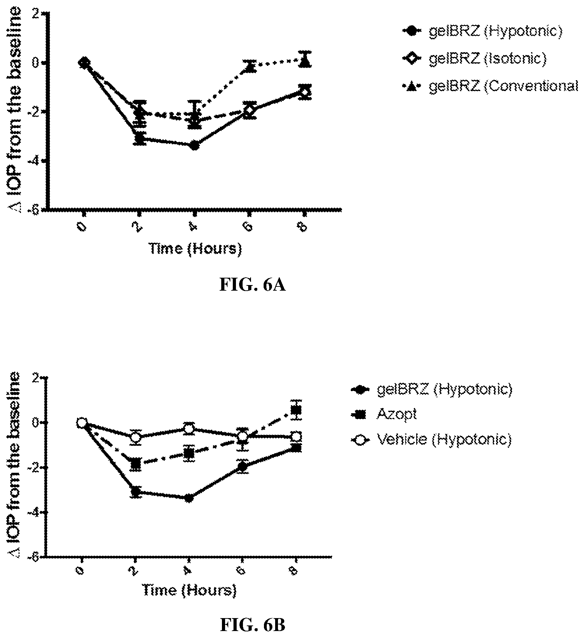

**Figures 6A and 6B** are line graphs showing changes in intraocular pressure as a function of time in hours with different treatments. **Figure 6A** a graph showing changes over the period of 8 hours in intraocular pressure when treated with gelBRZ formulated as either a conventional (18% F127) gelling material, or at the lower concentration (12% F127) formulated as either hypotonic or isotonic. **Figure 6B** is a graph showing changes in intraocular pressure when treated with hypotonic gelBRZ formulation, a commercial eye drop, AZOPT^{®}, or gelBRZ vehicle over the period of 8 hours.

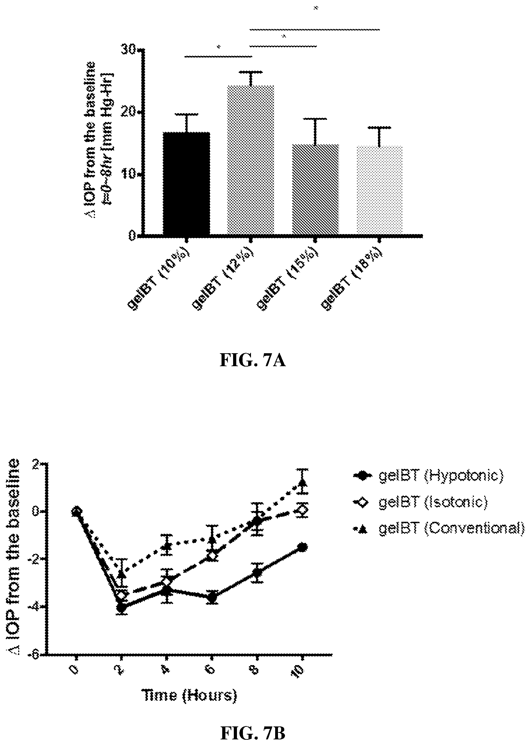

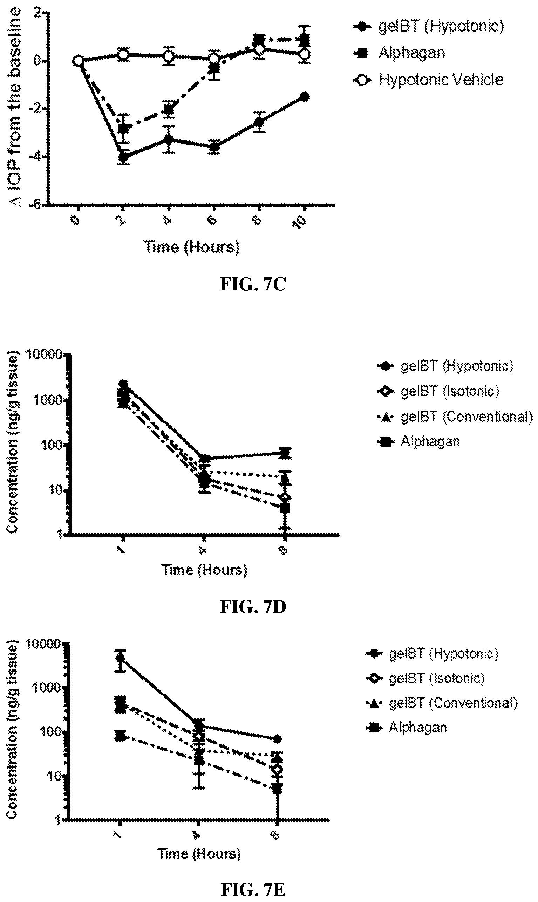

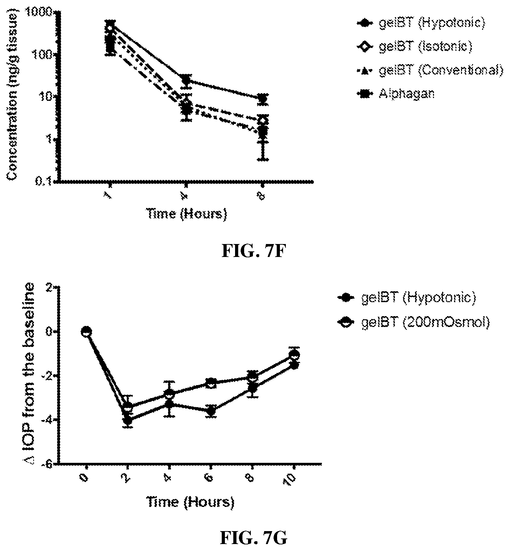

**Figures 7A** is a bar graph showing the cumulative change in IOP over 8 h after administration of brimonidine tartrate 0.15% in 10%, 12%, 15%, and 18% F127 (gelBT). **Figure 7B** is a line graph showing changes in intraocular pressure when treated with hypotonic gelBT formulation, isotonic gelBT (12% F127), or conventional gelBT (18% F127) in normotensive rabbits over a 10-hour period. **Figure 7C** is a line graph showing changes in intraocular pressure when treated with hypotonic gelBT formulation, the commercial 0.15% brimonidine tartrate eye drop (ALPHAGAN^{®}), or gelBT vehicle over the period of 10 hours. **Figures 7D-7F** show brimonidine levels in cornea (FIG.7D), conjunctiva (FIG.7E), and aqueous (FIG.7F) from rats 1, 4, and 8 hr after the last dose (dosing 2x daily for 5 days). **Figure 7G** is a line graph showing changes in intraocular pressure when treated with a mild hypotonicity (gelBT 200 mOsm) or hypotonic gelBT.

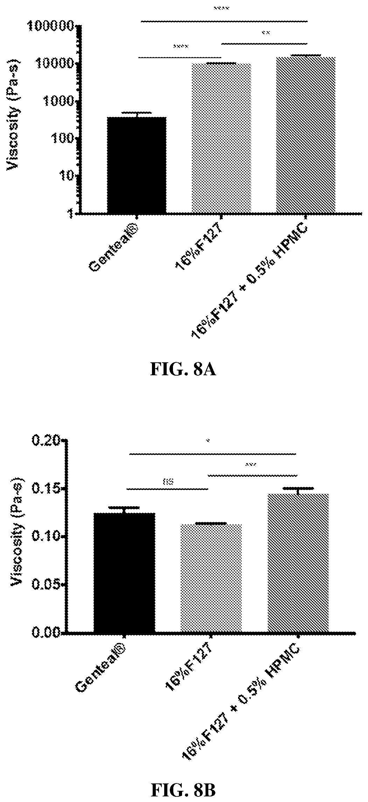

**Figure 8A** is a bar graph showing the viscosity under low/no shear of a standard lubricating eye drop (GENTEAL^{®}), 16% F127, and 16% F127 with 0.5% HPMC. **Figure 8B** shows that the shear thinning properties (decrease in viscosity when placed under shear) for GENTEAL^{®}), 16% F127, and 16% F127 with 0.5% HPMC.

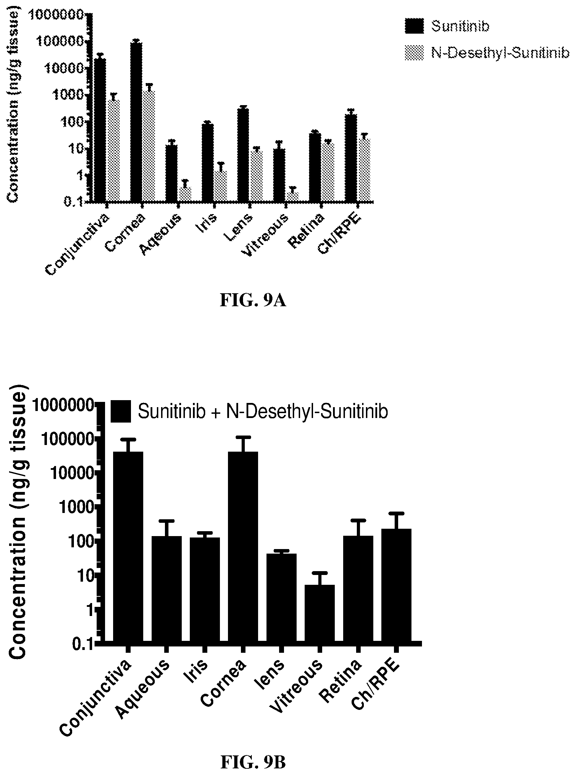

**Figures 9A** and **9B** are histograms showing concentration of sunitinib or N-desethyl sunitinib in various ocular tissues and fluids in dutch belted rabbits following daily topical dosing with sunitinib malate in the hypotonic gelling vehicle (gelSUN) for 14 days (FIG. 13A), and in farm pigs following daily topical dosing with sunitinib malate in the hypotonic gelling vehicle (gelSUN) for 5 days.

### DETAILED DESCRIPTION OF THE INVENTION

### I. DEFINITIONS

As generally used herein "pharmaceutically acceptable" refers to those compounds, materials, compositions, and/or dosage forms which are, within the scope of sound medical judgment, suitable for use in contact with the tissues of human beings and animals without excessive toxicity, irritation, allergic response, or other problems or complications commensurate with a reasonable benefit/risk ratio.

"Biocompatible" and "biologically compatible", as used herein, generally refer to materials that are, along with any metabolites or degradation products thereof, generally non-toxic to the recipient, and do not cause any significant adverse effects to the recipient. Generally speaking, biocompatible materials are materials which do not elicit a significant inflammatory, immune or toxic response when administered to an individual.

The terms "gel" and "hydrogel", as used herein, refers to a swollen, water-containing network of finely-dispersed polymer chains that are water-insoluble, where the polymeric molecules are in the external or dispersion phase and water (or an aqueous solution) forms the internal or dispersed phase. The chains can be chemically crosslinked (chemical gels) or physically crosslinked (physical gels). Chemical gels possess polymer chains that are connected through covalent bonds, whereas physical gels have polymer chains linked by non-covalent bonds or cohesion forces, such as Van der Waals interactions, ionic interaction, hydrogen bonding, or hydrophobic interaction.

The polymer chains are typically hydrophilic or contain hydrophilic polymer blocks. "Gel-forming polymers" is used to describe any biocompatible polymer, including homopolymers, copolymers, and combinations thereof, capable of forming a physical hydrogel in an aqueous medium when present at or above the critical gel concentration (CGC).

The "critical gel concentration", or "CGC", as used herein, refers to the minimum concentration of gel-forming polymer needed for gel formation, e.g. at which a solution-to-gel (sol-gel) transition occurs. The critical gel concentration can be dependent on a number of factors, including the specific polymer composition, molecular weight, temperature, and/or the presence of other polymers or excipients.

The term "thermosensitive gel-forming polymer" refers to a gel-forming polymer that exhibits one or more property changes with a change in the temperature. For example, some thermosensitive gel-forming polymers are water soluble below a certain temperature but become water insoluble as temperature is increased. The term "lower critical solution temperature (LCST)" refers to a temperature, below which a gel-forming polymer and solvent are completely miscible and form a single phase. For example, "the LCST of a polymer solution" means that the polymer is uniformly dispersed in a solution at that temperature (i.e., LCST) or lower, but aggregates and forms a second phase when the solution temperature is increased beyond the LCST.

"Hydrophilic," as used herein, refers to molecules which have a greater affinity for, and thus solubility in, water as compared to organic solvents. The hydrophilicity of a compound can be quantified by measuring its partition coefficient between water (or a buffered aqueous solution) and a water-immiscible organic solvent, such as octanol, ethyl acetate, methylene chloride, or methyl tert-butyl ether. If after equilibration a greater concentration of the compound is present in the water than in the organic solvent, then the compound is considered hydrophilic.

"Hydrophobic," as used herein, refers to molecules which have a greater affinity for, and thus solubility in, organic solvents as compared to water. The hydrophobicity of a compound can be quantified by measuring its partition coefficient between water (or a buffered aqueous solution) and a water-immiscible organic solvent, such as octanol, ethyl acetate, methylene chloride, or methyl tert-butyl ether. If after equilibration a greater concentration of the compound is present in the organic solvent than in the water, then the compound is considered hydrophobic.

As used herein, the term "treating" includes inhibiting, alleviating, preventing or eliminating one or more symptoms or side effects associated with the disease, condition, or disorder being treated.

The term "reduce", "inhibit", "alleviate" or "decrease" are used relative to a control, either no other treatment or treatment with a known degree of efficacy. One of skill in the art would readily identify the appropriate control to use for each experiment. For example a decreased response in a subject or cell treated with a compound is compared to a response in subject or cell that is not treated with the compound.

As used herein the term "effective amount" or "therapeutically effective amount" means a dosage sufficient to treat, inhibit, or alleviate one or more symptoms of a disease state being treated or to otherwise provide a desired pharmacologic and/or physiologic effect. The precise dosage will vary according to a variety of factors such as subject-dependent variables (*e.g*., age, immune system health, etc.), the disease or disorder, and the treatment being administered. The effect of the effective amount can be relative to a control. Such controls are known in the art and discussed herein, and can be, for example the condition of the subject prior to or in the absence of administration of the drug, or drug combination, or in the case of drug combinations, the effect of the combination can be compared to the effect of administration of only one of the drugs.

"Excipient" is used herein to include a compound that is not a therapeutically or biologically active compound. As such, an excipient should be pharmaceutically or biologically acceptable or relevant, for example, an excipient should generally be non-toxic to the subject. "Excipient" includes a single such compound and is also intended to include a plurality of compounds.

The term "osmolarity", as generally used herein, refers to the total number of dissolved components per liter. Osmolarity is similar to molarity but includes the total number of moles of dissolved species in solution. An osmolarity of 1 Osm/L means there is 1 mole of dissolved components per L of solution. Some solutes, such as ionic solutes that dissociate in solution, will contribute more than 1 mole of dissolved components per mole of solute in the solution. For example, NaCl dissociates into Na⁺ and Cl⁻ in solution and thus provides 2 moles of dissolved components per 1 mole of dissolved NaCl in solution. Physiological osmolarity is typically in the range of about 280 to about 310 mOsm/L.

The term "tonicity", as generally used herein, refers to the osmotic pressure gradient resulting from the separation of two solutions by a semipermeable membrane. In particular, tonicity is used to describe the osmotic pressure created across a cell membrane when a cell is exposed to an external solution. Solutes that can cross the cellular membrane do not contribute to the final osmotic pressure gradient. Only those dissolved species that do not cross the cell membrane will contribute to osmotic pressure differences and thus tonicity. The term "hypertonic", as generally used herein, refers to a solution with a higher concentration of solutes than is present on the inside of the cell. When a cell is immersed into a hypertonic solution, the tendency is for water to flow out of the cell in order to balance the concentration of the solutes. The term "hypotonic", as generally used herein, refers to a solution with a lower concentration of solutes than is present on the inside of the cell. When a cell is immersed into a hypotonic solution, water flows into the cell in order to balance the concentration of the solutes. The term "isotonic", as generally used herein, refers to a solution wherein the osmotic pressure gradient across the cell membrane is essentially balanced. An isotonic formulation is one which has essentially the same osmotic pressure as human blood. Isotonic formulations will generally have an osmotic pressure from about 250 to 350 mOsm.

### II. HYPOTONIC GEL-FORMING COMPOSITIONS

Hypotonic formulations of hydrogel forming polymers, in which the hydrogel forming polymers are (i) between greater than 12 and less than 24% Pluronic^{®} F98 in an aqueous excipient, or (ii) between 10 and 18% Pluronic^{®} F127, have been developed for enhanced delivery through mucus of therapeutic, diagnostic, prophylactic or other agents, to a mucosal or epithelial surface of the eye. The polymers are administered at a concentration less than their normal critical gelling concentration (CGC). A Poloxamer gel administered into the vagina or colorectum at a concentration equal to or above its CGC will form a "plug" of gel in the lumen. In contrast, fluid from a hypotonically-administered Poloxamer solution as defined in the claims, where the Poloxamer is at a concentration below its CGC, will be absorbed into mucosal tissues, thereby drawing the Poloxamer through the mucus gel toward the epithelium, thereby enhancing and facilitating delivery of agents to the body. As water is absorbed into the tissues, the Poloxamer becomes concentrated and gels near the epithelial tissue surface, thereby trapping drug molecules in a sustained-release gel on the tissue surface (rather than, *e.g*., in a gel that forms primarily in the lumen as occurs with traditional thermogelling methods whereby the gelling polymers are administered at a concentration at or above their CGC). Endogenous mucin glycopolymers affect the gelling properties of the hypotonic gelling agents, including the concentration of gelling agent needed to gel and the pore structure of the resulting gel/mucin mixture. After ocular application, the hypotonic gelling vehicles coat the epithelium, including the folds or inner eyelids.

In the case of ocular administration, the hypotonic gelling agent forms a uniform gel coating on the surface of the eye, rather than gelling in a bolus that is rapidly cleared away by blinking.

Gel-forming compositions that are capable of forming uniform gel coatings on epithelial surfaces but do not gel under storage conditions are described herein. The hypotonic formulations defined in the claims contain a gel forming polymer that is (i) between greater than 12 and less than 24% Pluronic^{®} F98 in an aqueous excipient, or (ii) between 10 and 18% Pluronic^{®} F127, and contain one or more additional excipients to form a pharmaceutically acceptable hypotonic formulation of the polymer suitable for delivery to the eye of an individual in need thereof, and one or more therapeutic, prophylactic, or diagnostic agents.

### A. Hydrogel-Forming Polymers

The hypotonic gel-forming compositions contain one or more gel-forming polymers as defined in the claims. Gel-forming polymers are utilized at a concentration below the normal critical gel concentration (CGC) of the polymer, *e.g.* the concentration at which the polymer solution would gel in a test tube when warmed to 37°C.

Thermosensitive (aka thermoresponsive) hydrogels are solutions that undergo sol-gel transitions when the following criteria are both met:
1) at or above the critical gelling concentration (CGC), and
2) at or above the critical gelling temperature.

Thermosensitive gelling agents (at or above their CGC) used for biomedical applications are liquid at room temperature, but form a gel at body temperature. The increase in temperature induces a rearrangement and alignment of the polymer chains, leading to gelation into a 3-dimensional structure. This phenomenon is generally governed by the ratio of hydrophilic to hydrophobic moieties on the polymer chain. A common characteristic is the presence of a hydrophobic methyl, ethyl, or propyl group. Thermosensitive polymers that fit these criteria can be administered topically in a hypotonic solution at a range of concentrations that is below its CGC to mucosal tissues to form a uniform gel coating *in vivo.*

Examples of thermosensitive gel formers that can be used include polyoxyethylene-polyoxypropylene-polyoxyethylene triblock copolymers such as, but not limited to, those designated by the CTFA names Poloxamer 407 (CAS 9003-11-6, molecular weight 9,840-14,600 g/mol, percentage of polyoxyethylene by weight approximately 70%; available from BASF as LUTROL^{®} F127) and Poloxamer 188 (CAS 9003-11-6, molecular weight 7680-9510 g/mol, percentage of polyoxyethylene by weight approximately 80%; available from BASF as LUTROL^{®} F68); Poloxamers are also known by the trade name PLURONIC^{®} *e.g*., PLURONIC^{®} F98 (CAS 9003-11-6, molecular weight 13000 g/mol, percentage of polyoxyethylene by weight approximately 80%; available from BASF); Tetronics tetra-functional block copolymers based on ethylene oxide and propylene oxide available from BASF as TETRONIC^{®}; poly(N,N-diethylacrylamide); poly(N,N-dimethylacrylamide); poly(N-vinylcaprolactam); poly(N-alkylacrylamide); poly(N-vinylalkylamide); poly(N-isopropyl acrylamide); polyethylene oxide methacrylate polymers; poly(lactic-co-glycolic acid) (PLGA)-polyethylene glycol triblock copolymers (PLGA-PEG-PLGA and PEG-PLGA-PEG); polycaprolactone (PCL)-polyethylene glycol triblock copolymers (PCL-PEG-PCL and PEG-PCL-PEG); chitosan; and combinations thereof.

The hydrogels can be formed from individual gel formers or as a combination of gel formers. For example, a poloxamer and another gel former (*e.g.*, a tetronic polymer) may be used in combination to attain the desired characteristics. In addition, various forms of the same gel former (*e.g*., Poloxamer 188 and Poloxamer 407) can be combined to attain the desired characteristics.

The polymer is provided in a concentration less than the concentration in aqueous solution that forms a gel in a test tube when heated to 37°C. The concentration must be sufficiently high, but below the CGC, for the epithelium to absorb enough fluid for the CGC to be reached *in vivo,* so gelation can occur preferentially on / near the mucosal epithelial surface. The range of time that it takes for gelation to occur depends on the mucosal surface (the capacity and rate of water absorption), the tonicity of the solution administered (more hypotonic solutions will drive more rapid fluid absorption), and the concentration of polymer administered (if the polymer concentration is too low, not enough fluid absorption will occur to concentrate the polymer to its CGC). However, gelation generally occurs within 1 h in the vagina and colorectum.

The concentration of the polymer and the presence of additional components such as the endogenous mucins affect coverage and rate and degree of gelling. 18% F127 gel mixed with purified pig gastric mucins (1%) or human cervicovaginal mucus (1:1 ratio) does not trap virus-sized (~100 nm) nanoparticles (polyethylene glycol coated polystyrene nanoparticles, PSPEG) as effectively as 18% F127 gel alone. In contrast, 24% F98 gel more effectively trapped PSPEG particles when mixed with mucins or human cervicovaginal mucus. However, *in vivo* viral trapping with hypotonic gelling agents was more effective at trapping viruses, including human immunodeficiency virus (HIV, ~120 nm) and herpes simplex virus (HSV, ~180 nm). Administration of hypotonic solution containing 18% F98, having a CGC of 24%, results in effective trapping of subsequently administered HIV in the vagina. Similarly, hypotonic solution containing 10% and 15% F127, having a CGC of 18%, were also effective in decreasing the MSD of HIV, indicating trapping. Additionally, both 15% F127 and 18% F98 reduced the diffusion of subsequently administered HSV in mouse vaginal mucus. The distribution of the individual virus MSD at a time scale of 1 s illustrated that the trapping (shift to the left) of the viruses was more uniform in the gel formed by the hypotonic 18% F98 vehicle compared to 15% F127. In additional tests of viral trapping by hypotonic gelling agents in the colorectum, it was found that 12% F98 (CGC 24%) did not effectively trap PSPEG nanoparticles administered 30 mins after the gelling vehicle, though 18% F98 was effective at trapping PSPEG nanoparticles in the mouse colorectum. Importantly, these examples illustrate differences in the gels that form when the hypotonic gelling agents are administered to different mucosal surfaces, and in this case, mix with vaginal mucus compared to colorectal mucus prior to gelling.

### B. Hypotonic Carriers

The gel-forming compositions include a hypotonic carrier. The hypotonic carrier will typically be a biocompatible carrier that preferably causes little to no signs of irritation when administered to human subjects. The carrier can be naturally occurring or non-naturally occurring including both synthetic and semi-synthetic carriers. Preferred carriers are water-based. Other solutions, including sugar-based (*e.g*. glucose, mannitol) solutions and various buffers (phosphate-buffers, tris-buffers, HEPES), may also be used.

When hypotonic solutions are applied to an epithelial surface, a fluid shift occurs and water is moved into the epithelial tissue. This can cause swelling of the epithelial cells. In some cases, when the osmotic pressure difference is too large, the epithelial cells may burst causing tissue irritation or disruption of the epithelial membrane.

Hypotonic solution refers to a solution that causes water absorption by the epithelial surface to which it is administered. Examples of hypotonic solutions include, but are not limited to, Tris[hydroxylmethyl]-aminomethane hydrochloride (Tris-HCl, 10-100 mM, pH. 6-8), (4-(2-hydroxyethyl)-1-piperazineethanesulfonic acid (HEPES, 10-100 mM, pH 6-8) and dilute solutions of PBS, such as a solution containing 0.2 grams KCl, 0.2 grams KH₂PO₄, 8 grams NaCl, and 2.16 grams Na₂HPO₄*7H₂O in 1000 ml H₂O.

Hypotonic carriers cause dissolved gel-forming polymers to concentrate at an epithelial surface, resulting in uniform gel formation on the surface. The hypotonic carrier usually contains water as the major component. The hypotonic carrier can be water, although mixtures of water and a water-miscible organic solvent can also be used. Suitable water-miscible organic solvents include alcohols, such as ethanol, isopropanol; ketones, such as acetone; ethers such as dioxane; and esters such as ethyl acetate.

The hypotonic carrier can be distilled water containing one or more osmolarity modifying excipients. Sodium chloride is the excipient that is most frequently used to adjust osmolarity if a solution is hypotonic. Other excipients used to adjust hypotonic solutions include glucose, mannitol, glycerol, propylene glycol and sodium sulfate. Osmolarity modifying excipients can include pharmaceutically acceptable salts such as sodium chloride, sodium sulfate, potassium chloride, and other salts to make buffers such as dibasic sodium phosphate, monobasic potassium phosphate, calcium chloride, and magnesium sulfate. Other excipients used to adjust tonicity can include glucose, mannitol, glycerol, or propylene glycol.

The hypotonic carrier can have any osmolarity less than the effective isotonic point (the concentration at which fluid is neither absorbed nor secreted by the underlying tissues) at that mucosal surface. The isotonic point varies for different mucosal surfaces and different buffers, depending on active ion transport at that epithelial surface; e.g. we have found the isotonic point in the vagina for sodium-based solutions to be about 300 mOsm/L, but in the colorectum, it is about 450 mOsm/L. In some embodiments the solution has a tonicity from 50 mOsm/L to 280 mOsm/L, from 100 mOsm/L to 280 mOsm/L, from 150 mOsm/L to 250 mOsm/L, from 200 mOsm/L to 250 mOsm/L, from 220 mOsm/L to 250 mOsm/L, from 220 mOsm/L to 260 mOsm/L, from 220 mOsm/L to 270 mOsm/L, or from 220 mOsm/L to 280 mOsm/L.

The hypotonic carrier can include one or more pharmaceutically acceptable acid, one or more pharmaceutically acceptable base, or salts thereof. Pharmaceutically acceptable acids include hydrobromic, hydrochloric, and sulphuric acids, and organic acids, such as methanesulphonic acids, tartaric acids, and malcic acids. Pharmaceutically acceptable bases include alkali metal (*e.g*. sodium or potassium) and alkali earth metal (*e.g*. calcium or magnesium) hydroxides and organic bases such as pharmaceutically acceptable amines. The hypotonic carrier can include pharmaceutically acceptable buffers such as citrate buffers or phosphate buffers.

### C. Additional Agents

The hypotonic formulations defined in the claims contain one or more therapeutic agents, prophylactic agents, diagnostic agents, and/or nutraceuticals for use in treating, preventing or diagnosing an eye disease. A biologically active agent is a substance used for the treatment (*e.g*., therapeutic agent), prevention (*e.g.*, prophylactic agent), diagnosis (*e.g.,* diagnostic agent), or to effect a cure or mitigation of disease or illness, alter the structure or function of the body, or pro-drugs, which become biologically active or more active after they have been placed in a predetermined physiological environment. These may be small-molecule drugs ((*e.g*., molecular weight less than 2000, 1500, 1000, 750, or 500 atomic mass units (amu)), peptides or proteins, sugars or polysaccharides, nucleotides or oligonucleotides such as aptamers, siRNA, and miRNA, lipids, glycoproteins, lipoproteins, or combinations thereof.

The agents can include one or more of those described in Martindale: The Complete Drug Reference, 37th Ed. (Pharmaceutical Press, London, 2011).

In one embodiment, the agent to be delivered is poorly soluble in water, but soluble in the carrier containing the gelling polymer(s). In other embodiment, the agents are water-soluble. Data also show that benefit is obtained with water soluble drugs, for example, Brimonidine tartrate, which is soluble up to approximately 1 mg/mL.

The hypotonic gel-forming formulations can contain a therapeutically effective amount of a therapeutic agent to treat, inhibit, or alleviate one or more symptoms of a disease state being treated. The hypotonic gel-forming compositions can contain an effective amount of a prophylactic agent to prevent one or more symptoms of a disease or disorder.

Agents may be anti-infective (antibiotics, antivirals, antifungals), for treatment of eye disorders (glaucoma, dry eye), anti-inflammatories, inhibit neovascularization, fibrosis).

Exemplary agents include brinzolamide, cyclosporine A, brimonidine tartrate, moxifloxacin, budesonide, sunitinib, and acriflavine.

Examples of useful proteins include hormones such as insulin, growth hormones including somatomedins, and reproductive hormones. Examples of useful drugs include neurotransmitters such as L-DOPA, antihypertensives or saluretics such as Metolazone from Searle Pharmaceuticals, carbonic anhydrase inhibitors such as Acetazolamide from Lederle Pharmaceuticals, insulin like drugs such as glyburide, a blood glucose lowering drug of the sulfonylurea class, synthetic hormones such as Android F from Brown Pharmaceuticals and TESTRED^{®} (methyltestosterone) from ICN Pharmaceuticals. Representative antiproliferative (anti-cancer or endometriosis) agents include, but are not limited to, alkylating agents (such as cisplatin, carboplatin, oxaliplatin, mechlorethamine, cyclophosphamide, chlorambucil, dacarbazine, lomustine, carmustine, procarbazine, chlorambucil and ifosfamide), antimetabolites (such as fluorouracil (5-FU), gemcitabine, methotrexate, cytosine arabinoside, fludarabine, and floxuridine), antimitotics (including taxanes such as paclitaxel and decetaxel and vinca alkaloids such as vincristine, vinblastine, vinorelbine, and vindesine), anthracyclines (including doxorubicin, daunorubicin, valrubicin, idarubicin, and epirubicin, as well as actinomycins such as actinomycin D), cytotoxic antibiotics (including mitomycin, plicamycin, and bleomycin), topoisomerase inhibitors (including camptothecins such as camptothecin, irinotecan, and topotecan as well as derivatives of epipodophyllotoxins such as amsacrine, etoposide, etoposide phosphate, and teniposide), and combinations thereof. Other suitable anti-cancer agents include angiogenesis inhibitors including antibodies to vascular endothelial growth factor (VEGF) such as bevacizumab (AVASTIN^{®}), other anti-VEGF compounds; thalidomide (THALOMID^{®}) and derivatives thereof such as lenalidomide (REVLIMID^{®}); endostatin; angiostatin; receptor tyrosine kinase (RTK) inhibitors such as sunitinib (SUTENT^{®}); tyrosine kinase inhibitors such as sorafenib (NEXAVAR^{®}), erlotinib (TARCEVA^{®}), pazopanib, axitinib, and lapatinib; transforming growth factor-α or transforming growth factor-β inhibitors, and antibodies to the epidermal growth factor receptor such as panitumumab (VECTIBIX^{®}) and cetuximab (ERBITUX^{®}).

For imaging, radioactive materials such as Technetium99 (^{99m}Tc) or magnetic materials such as labelled-Fe₂O₃ could be used. Examples of other materials include compounds which are radioopaque.

### III. Methods and Preparations of Hypotonic Gel-forming Compositions

Drug solubilization provides potential advantages that include enhanced physical stability upon storage, increased drug penetration into the body, and a more reproducible drug dose when administered to a patient.

Water insoluble drugs are especially challenging to deliver to mucosal surface, such as that of the eye, gastrointestinal tract, female reproductive tract, airways, *etc.* due to lack of absorption. The formulations are particularly suited for delivering therapeutic agents that are poorly water-soluble. Water-soluble drugs are also challenging to deliver to a mucosal surface in a sustained fashion. Thus, the gelling material can also be used as a vehicle to improve the mucosal delivery of water-soluble drugs by, for example, providing more sustained drug absorption which can reduce side effects and provide more prolonged efficacy.

Further, mucosal surfaces are rapidly cleared and renewed as a normal defense mechanism from infections and foreign particulates. Improving drug solubilization can improve mucosal penetration, and improving distribution, penetration, and retention of hydrophobic drugs is a promising strategy for improving therapeutic effects. The following examples demonstrate the solubilization of various drugs and drug complexes with low water solubility into materials that also have thermosensitive gelling properties.
**Figures 1-4** depict schemes for achieving direct drug solubilization of hydrophobic drugs and drug complexes into the gelling material.
**Figure** 1 is a scheme for solubilization of hydrophobic drugs *(e.g.* cyclosporine A or budesonide) by a solvent evaporation method.
**Figure** 2 is a scheme for solubilization of hydrophobic drugs *(e.g.* moxifloxacin) by a bead milling method.
**Figure** 3 is a scheme for solubilization of hydrophobic drugs *(e.g.* cyclosporine A or brimonidine) by a dialysis method.
**Figure** 4 is scheme for solubilization of hydrophobic drugs *(e.g.* brinzolamide) by a solvent evaporation method.

Formulations can be prepared as described in the examples below.

The formulations can be prepared as liquids for administration. The gel forming liquid or polymer solubilizes insoluble drugs by forming micelles. Powder can be made by freeze-drying and reconstituted at the time of use.

The formulations may also include pharmaceutically acceptable diluents, preservatives, solubilizers, stabilizer, emulsifiers, adjuvants and/or carriers. Stabilizers such as SPAN^{®} 20 (sorbitan laurate, CAS Number 1338-39-2) may facilitate dissolution and prevent re-aggregation. Other exemplary stabilizers include polysorbates or TWEENS^{®}, e.g., poly-sorbate 20, polysorbate 60, polysorbate 65 and polysorbate 80, and polyglycerol esters (PGE) , polyoxyethylene alkyl ethers, poloxyl stearates, fatty acids (e.g. oleic acid) and propylene glycol monostearate (PGMS). In some cases, the composition includes one or more stabilizers.

Increased viscosity under no shear may lead to increased residence time on the mucosal surface of the eye, while shear thinning when blinking maximizes comfort and lubrication. Thus, gelling materials with these properties may have favorable lubricating properties on the surface of the eye while resisting clearance upon blinking. In some examples, one or more polymer materials are incorporated into the gelling vehicle for increasing viscosity under no shear and/or shear thinning when blinking. In one example, hydroxypropyl methylcellulose (HPMC) is incorporated into F127 solutions for achieving these desired properties. In some examples, shear thinning polymers in the concentration range in which they conventionally demonstrate shear thinning properties are also included in the composition. Exemplary shear thinning polymers include cellulose derivatives such as methyl cellulose, carboxymethylcellulose, hydroxypropyl cellulose, methylcellulose, *etc.*

Dosage formulations will typically be prepared as single or multiple liquid or dry dosage units in an appropriate applicator. A person of ordinary skill in the art will be aware of many options for drug storage and application, such as dual chambered devices that may be used to keep various components separate during storage. Multiple dosage units will typically include a barrel loaded with powder, and a plunger having dosage increments thereon. These will typically be sterilized and packaged in sealed, sterile packaging for storage and distribution. See also Remington: The Science and Practice of Pharmacy, 22nd Edition.

Dosage unit administrators may be designed to fit the anatomic location to which drug is to be delivered, such as intrarectally, intravaginally, intranasally, or intrabuccally.

### IV. Methods of Administering Hypotonic Gel-forming Compositions

The hypotonic gel-forming compositions are applied to a mucosal or epithelial surface of the eye to form a gel. Preferably, the formulations are applied as a liquid to a mucosal coating on an epithelial surface of the eye of a subject in need of a therapeutic, prophylactic, diagnostic, or nutritional effect. The gel-forming composition can be applied in any number of ways known to the skilled artisan as long as the hypotonic solution, or reagents forming the hypotonic solution, contacts the surface.

By applying the gel-forming compositions as a hypotonic formulation, water is absorbed into the epithelial tissue. Water absorption provides for concentration of the gel-forming polymer at the surface, resulting in uniform gel formation at the surface. The gel can act as a barrier, reservoir, or combination thereof. Agents or excipients in the gel-forming composition can become entrapped in the gel and can be released at or into the surface.

The epithelial surfaces are ocular surfaces.

In some examples, the hypotonic gel-forming compositions retain an effective concentration of one or more active agents at one or more mucosal surfaces for an extended period of time, for example, more than 6 hours, more than 12 hours, more than 1 day, more than 2 days, more than 3 days, more than 4 days, more than 5 days, more than 6 days, or more than a week.

In some cases, the hypotonic gel-forming compositions increase the concentration of one or more active agents at or near the site of application by 2-fold, 3-fold, 4-fold, 5-fold, 6-fold, 7-fold, 8-fold, 9-fold, 10-fold, or more than 10-fold compared to active agents delivered without gel-forming vehicles, for example, in saline solution. When the hypotonic gel-forming compositions are applied to the eye, the mucosal sites with increased concentration of active agents include one or more of cornea, aqueous humor, sclera, conjunctiva, iris, lens, retina, and retinal pigment epithelium.

In some examples, the hypotonic gel-forming compositions deliver active agents (*e.g*., acriflavine and sunitinib malate) to retina and/or choroid in an amount effective to reduce retinal and/or choroidal neovascularization by 10%, 20%, 30%, 40%, 50%, or more than 50% compared to active agents delivered without gel-forming vehicles, for example, in saline solution.

In other examples, the hypotonic gel-forming compositions deliver active neuroprotective agents (*e.g*., sunitinib malate) to the retina in an amount effective to increase the survival of retinal ganglion cells following optic nerve injury, and/or to increase the expression of γ-synuclein and/or βIII tubulin in retinal ganglion cells following optic nerve injury by 2-fold, 3-fold, 4-fold, 5-fold, or more than 5-fold compared to active agents delivered without gel-forming vehicles, for example, in saline solution.

In further examples, the hypotonic gel-forming compositions deliver active agents (*e.g*., brinzolamide) to the eye in an amount effective to lower intraocular pressure (IOP) by 10%, 20%, 30%, 40%, 50%, or more than 50% compared to those delivered without gel-forming vehicles, for example, in saline solution within less than 2 hours, 4 hours, 6 hours, 8 hours, 10 hours, 12 hours, or 24 hours.

In other examples, the hypotonic gel-forming compositions deliver active agents (*e.g.*, Cyclosporine A) to the eye in an amount effective to increase tear production by 10%, 20%, 30%, 40%, 50%, or more than 50% compared to those delivered without gel-forming vehicles, for example, in saline solution within less than 2 hours, 4 hours, 6 hours, 8 hours, 10 hours, 12 hours, or 24 hours.

The present invention will be further understood by reference to the following non-limiting examples.

### Examples

### Example 1: Cyclosporine A Eye Drops

Rapid drug elimination from the ocular surface is a major obstacle for topical drug delivery, and as a result, many eye drops are prescribed for several times daily application.

### Materials and Methods

The first example embodies the solubilization of cyclosporine A (used for dry eye) in the thermosensitive gelling vehicle (gelCsA) prepared using the method shown schematically in **Figure 3****.** Cyclosporine A and Pluronic^{®} F127 (10-18% w/w) were co-dissolved in DMSO and dialyzed against water balanced with salts (sodium chloride, final osmolality 200 mOsm) for 2 days to remove DMSO. CsA concentration in all the formulations were 0.05%. Tear production was measured using Schirmer's strips held in contact with the ocular surface. Drug concentrations were measured using LC-MS.

### Results

It has been reported in the literature that cyclosporine increases tear production in healthy animal eyes. Figure 5A shows cumulative increase in tear production induced by cyclosporine in healthy rabbit eyes for 12 h after a single drop of Cyclosporine A formulated in various concentrations of Pluronic^{®} F127 including 10%, 12%, 15%, and 18% w/w. The hypotonic 12% F127 vehicle for cyclosporine provided the largest increase in tear production.

Figure 5B shows a graph of the tear production in the healthy rabbit eye 10 h after each of 5 daily doses of gelCsA, the gel vehicle (containing no drugs), RESTASIS^{®}, the RESTASIS^{®} vehicle (Refresh Endura), or no treatment (Untreated). Only in the case of gelCsA is an increase in tear production due to cyclosporine delivery observed 10 h after eye drops were dosed. This further demonstrates the potential for gelCsA to be dosed once per day, whereas RESTASIS^{®} is prescribed twice per day.

Figures 5C and D show cyclosporine levels in rat cornea and conjunctiva tissue after administering various cyclosporine eye drops, demonstrating that the hypotonic gelCsA provides superior cyclosporine delivery compared to the isotonic and conventional gelling vehicles, as well as RESTASIS^{®}.

### Example 2: Brinzolamide Drops for Glaucoma Therapy

### Materials and Methods

Brinzolamide is used to lower intraocular pressure (IOP) as a glaucoma therapy. Brinzolamide was formulated in the gelling vehicle (gelBRZ) using the method of **Figure 4****.** Pluronic^{®} F127 ranged from 10-18% and salts were added to adjust osmolality up to isotonic (300 mOsm/kg) when required. The effect of the vehicle itself (gelBRZ vehicle) and brinzolamide drops on IOP in normotensive rabbits was tested after administering single eye drop with 50uL at 10mg/mL.

### Results

Figure 6A is a graph showing the gelBRZ formulated as either a conventional (18% F127) gelling material, or at the lower concentration (12% F127) formulated to be either hypotonic or isotonic. Figure 6B shows that the gelBRZ vehicle had no effect on IOP of the treated or contralateral eye in normotensive rabbits. It further compares the hypotonic gelBRZ formulation to the commercial eye drop, AZOPT^{®} (a clinical brinzolamide ophthalmic suspension of 1% brinzolamide). A single dose of 1% brinzolamide had a more pronounced effect on IOP lowering for up to 8 h after treatment when delivered as gelBRZ (12%, hypotonic) compared to AZOPT^{®}. Further, hypotonic gelBRZ (35 mOsm) lowered IOP more than isotonic gelBRZ (300 mOsm) at early time points up to 4 h.

### Example 3: Brimonidine Tartrate Ophthalmic Formulation

### Materials and Methods

Brimonidine tartrate is a water-soluble drug that can be directly dissolved into the gelling vehicle (gelBT). It was first tested whether there was an optimal F127 concentration for administering the brimonidine tartrate in a hypotonic gelling vehicle to the eye, as measured by reduction in IOP in normotensive rabbits. IOP reduction was then compared for F127 concentrations ranging from 12-18% in hypotonic (no salt) and isotonic (salt to adjust to 300 mOsm/kg) in normotensive rabbits. Drug concentrations were measured by LC-MS.

### Results

Figures 7A shows the cumulative change in IOP over 8 h after administration of brimonidine tartrate 0.15% in F127 (10-18%) (gelBT). The cumulative decrease in IOP in normotensive rabbits over 8 h after a single drop of hypotonic gelBT was highest for gelBT containing 12% F127. Figure 7B shows hypotonic gelBT was more effective in lowering IOP in normotensive rabbits over an 8 h period compared to isotonic (12% F127) and conventional (18% F127) eye drops. Figure 7C shows the gelBT vehicle had no effect on IOP, and hypotonic gelBT was more effective than the commercial Alphagan eye drop at lowering IOP over 8 h. Figures D-F show BT levels in cornea, conjunctiva, and aqueous from rats 1, 4, and 8 h after the last dose (dosing 2x daily for 5 days). Drug levels were higher for hypotonic gelBT than all other formulations. Figure 7G shows that even a mild hypotonicity (200 mOsm) was as effective in lowering IOP for gelBT.

### Example 4: Incorporation of Polymer Materials into Gelling Vehicle

### Materials and Methods

Polymer materials were incorporated into the gelling vehicle, hydroxypropyl methylcellulose (HPMC) into F127 solutions, for shear thinning properties, which can be advantageous in the case of blinking (ocular administration) or lubrication (rectal administration). It also may increase the viscosity of the gel at rest (under no shear) and improve the retention time at mucosal surfaces.

### Results

As shown in Figure 8A, addition of 0.5% HPMC to F127 increases the viscosity under low/no shear, which is already thousands-fold higher than a standard lubricating eye drop (GENTEAL^{®}). Figure 8B shows that the shear thinning properties (decrease in viscosity when placed under shear) are similar for F127 solutions containing HPMC compared to standard lubricating eye drops (GENTEAL^{®}). Thus, addition of hydroxypropyl methylcellulose (HPMC) to F127 solutions increases the viscosity under low/no shear, while maintaining the low viscosity observed in standard lubricating eye drops (*e.g.* GENTEAL^{®}) under high shear (similar to shear rate when blinking).

Increased viscosity under no shear may lead to increased residence time on the mucosal surface of the eye, while shear thinning when blinking maximizes comfort and lubrication. Thus, such gelling materials may have favorable lubricating properties on the surface of the eye while resisting clearance upon blinking. Materials include shear thinning polymers in the concentration range in which they conventionally demonstrate shear thinning properties, such as cellulose derivatives including methyl cellulose, carboxymethylcellulose, hydroxypropyl cellulose, methylcellulose, *etc.*

### Example 5: No Ocular Irritation in the Presence of Hypotonic Gelling Vehicles with Different Osmolalities

New Zealand white rabbits were dosed topically with 50uL per eye drop twice per day for 5 weeks to assess potential ocular irritation. Test agents included 12% F127 solutions containing sodium chloride to produce final osmolalities ranging from no added salt up to 300 mOsm/kg. The negative control was balanced salt solution (BSS), and there was a group a animals receiving no treatment. There was no difference in corneal staining (lisamine green 1%) or blink rate (number of blinks in 3 min) for any formulation compared to untreated animals.

### Example 6: Drug Delivery To Retina/Choroid Through Topical Administration

C57/B6 mice where used to test the topical delivery of sunitinib malate (5uL at 4mg/mL) and acriflavine (5uL at 5mg/mL) in preventing choroidal neovascularization (CNV) in mice after laser induced rupture of Bruch's membrane in three locations. After laser, both drugs either dissolved in 12% F127 or a aqueous vehicle control (saline or water), were dosed daily for 7 days. Only the formulations containing 12% F127 were efficacious in suppressing neovascularization in the choroid.

### Example 7: Melanin-binding Prolonged Therapeutic Effects of Topically Dosed Sunitinib in Hypotonic Gelling Vehicle Following Optical Nerve Injury

Rats were dosed topically with sunitinib malate (5uL at 4mg/mL) in 12% F127 daily for 5 days unilaterally. Then, the optic nerve head was exposed and crushed unilaterally and the contralateral eye was used as the control for comparison. 14 days after crushing the nerve (without additional topical dosing), the tissues were isolated for qPCR for genes expressed by retinal ganglion cells (RGCs). This dosing scheme led to protection of RGCs in pigmented rats (Brown Norway) and not non-pigmented rats (Wistar), suggesting that binding to melanin in the eye led to a prolonged therapeutic effect.

### Example 8: Treatment Using Hypotonic Gelling Vehicle in Optical Nerve Injury

Pigmented rats (Brown Norway) were dosed topically (5uL at 4mg/mL) with sunitinib malate in 12% F127 or in saline once weekly unilaterally. The optic nerve head was exposed and crushed unilaterally on Day 8 and the contralateral eye was used as the control for comparison. 14 days after crushing the nerve (Day 22), the tissues were isolated for qPCR for genes expressed by retinal ganglion cells (RGCs). Protection of RGCs was only observed when sunitinib malate was dosed in 12% F127, but not when sunitinib malate was dosed in saline.

### Example 9: Ocular Tissue Distribution of Therapeutic Agents Delivered in Hypotonic Gelling Vehicles

### Materials and Methods

Sunitnib malate was dissolved in 12% F127 at a concentration of 4 mg/ml and dosed topically to pigmented (Dutch belted) rabbits and pigmented pigs (juvenile farm pigs) once per day for a total of 14 days or 5 days, respectively. Dosing in both rabbits and pigs occurred without anesthesia and 50uL or 50~100uL of an eye drop was administered, respectively. However, dosing in pigs occurred while distracted with food. Tissues were collected 6 h after the final dose for rabbits and 1 h after the final dose for pigs. The drug concentrations were measured by LC-MS.

### Results

Sunitinib malate was dosed topically in the hypotonic gelling vehicle was dosed topically to (FIG.9A) dutch belted rabbits daily for 14 days and (FIG.9B) farm pigs daily for 5 days. Tissues were isolated and sunitinib and primary metabolite (N-desethyl sunitinib) levels quantified in various ocular tissues and fluids. Therapeutically relevant levels of drug were found in the anterior and posterior segments.

Unless defined otherwise, all technical and scientific terms used herein have the same meanings as commonly understood by one of skill in the art to which the disclosed invention belongs.

Those skilled in the art will recognize, or be able to ascertain using no more than routine experimentation, many equivalents to the specific embodiments of the invention described herein.

## Claims

1. A hypotonic formulation for use in treating, preventing or diagnosing an eye disease comprising administering to a mucosal or epithelial surface of the eye a formulation comprising
a therapeutic, prophylactic, nutraceutical or diagnostic agent for the treatment of an eye disease,
a gel-forming polymer for application to the eye, formulated so that it is at a concentration below the critical gel concentration (CGC) of the polymer under isotonic conditions and a temperature between 25 to 37°C at the time of administration, and increases in concentration as water is absorbed from the formulation to or above said CGC, thereby forming a gel on the mucosal or epithelial surface, and
excipients to form a pharmaceutically acceptable hypotonic formulation of the polymer suitable for delivery to the eye of an individual in need thereof,
wherein the formulation retains an effective concentration of said agent at the mucosal or epithelial surface of the eye,
wherein the gel-forming polymer is (i) between greater than 12 and less than 24% poloxamer 288 (Pluronic^{®} F98) in an aqueous excipient, or (ii) is between 10 and 18% poloxamer 407 (Pluronic^{®} F127).

2. The formulation for use of claim 1 in dry or liquid form.

3. The formulation for use of any one of claims 1-2, wherein the gel-forming polymer is a thermosensitive gel-forming polymer.

4. The formulation for use of claim 3, wherein the thermosensitive gel-forming polymer has a lower critical solution temperature that is below 30°C, preferably below 21°C.

5. The formulation for use of any one of claims 1-4 wherein the polymer is a poloxamer.

6. The formulation for use of any one of claims 1-5, wherein the polymer in combination with excipient forms a gel on a mucosal or epithelial surface of the eye.

7. The formulation for use of any one of claims 1-6 wherein the agent is water-soluble, or wherein the agent is poorly water-soluble.

8. The formulation for use of any one of claims 1-6 wherein the agent is hydrophobic.

9. The formulation for use of any one of claims 1-8 wherein the hypotonic formulation has a tonicity of from 50 mOsm/L to 280 mOsm/L.

10. The formulation for use of any one of claims 1-9, comprising water and a water-miscible organic solvent, which may be an alcohol, ketone, ether or ester, optionally wherein:
(i) the alcohol is ethanol or isopropanol;
(ii) the ketone is acetone;
(iii) the ether is dioxane; or
(iv) the ester is ethyl acetate.

11. The formulation for use of any one of claims 1-10, wherein the formulation releases the therapeutic, prophylactic, diagnostic or nutraceutical agent at the epithelial surface over a period of at least 12 hours, or over a period of at least 24 hours.

12. The formulation for use of any one of claims 1-11, wherein the gel-forming polymer forms a uniformly thick layer at the time of administration onto the epithelial surface.

13. The formulation for use of any one of claims 1-12 for administration in the form of a dry powder, gel, or liquid, optionally wherein the formulation is provided in a single or multiple dosage unit for administration.

14. The formulation for use of any one of claims 1-13 wherein (i) the agent is a protein or peptide, small molecule, sugar or polysaccharide, lipid, glycolipid, glycoprotein, nucleic acid, oligomer or polymer thereof, or small molecule; or
(ii) the agent is selected from the group consisting of steroids, glaucoma agents, tyrosine kinase inhibitors, immunosuppressive agents, anti-fibrotic agents, anti-infectives, hormones and chemotherapeutic agents.

15. A hypotonic formulation as defined in any one of claims 1-14 for use in medicine, wherein the formulation is administered to a site in need thereof and said site is a mucosal or epithelial surface of the eye.

16. A dry dosage form comprising a poorly water-soluble therapeutic, prophylactic, nutraceutical or diagnostic agent and a freeze-dried powder suitable for reconstitution to form a hypotonic formulation as defined in any one of claims 1-14 at the time of use.

17. The hypotonic formulation for use of any one of claims 1-14, the hypotonic formulation for use of claim 15, or the dry dosage form of claim 16, wherein the mucosal or epithelial surface of the eye is one or more of cornea, aqueous humor, sclera, conjunctiva, iris, lens, retina and retinal pigment epithelium.

## Patentansprüche

1. Hypotonische Formulierung zur Verwendung bei der Behandlung, Vorbeugung oder Diagnose einer Augenkrankheit, umfassend die Verabreichung einer Formulierung an eine Schleimhaut- oder Epitheloberfläche des Auges, umfassend
ein therapeutisches, prophylaktisches, nutrazeutisches oder diagnostisches Mittel für die Behandlung einer Augenkrankheit,
ein gelbildendes Polymer zur Anwendung am Auge, das so formuliert ist, dass es unter isotonischen Bedingungen und einer Temperatur zwischen 25 und 37 °C zum Zeitpunkt der Verabreichung in einer Konzentration unterhalb der kritischen Gelkonzentration (CGC) des Polymers vorliegt und in seiner Konzentration zunimmt, wenn Wasser aus der Formulierung bis zu oder oberhalb der CGC absorbiert wird, wodurch ein Gel auf der Schleimhaut- oder Epitheloberfläche gebildet wird, und
Hilfsstoffe, um eine pharmazeutisch unbedenkliche hypotonische Formulierung des Polymers zu bilden, die für die Verabreichung an das Auge eines Individuums, das diese benötigt, geeignet ist,
wobei die Formulierung eine wirksame Konzentration des Mittels an der Schleimhaut- oder Epitheloberfläche des Auges zurückhält,
wobei das gelbildende Polymer (i) zwischen mehr als 12 und weniger als 24 % Poloxamer 288 (Pluronic^{®} F98) in einem wässrigen Exzipienten ist, oder (ii) zwischen 10 und 18 % Poloxamer 407 (Pluronic^{®} F127) ist.

2. Formulierung zur Verwendung nach Anspruch 1 in trockener oder flüssiger Form.

3. Formulierung zur Verwendung nach einem der Ansprüche 1 bis 2, wobei das gelbildende Polymer ein thermosensitives gelbildendes Polymer ist.

4. Formulierung zur Verwendung nach Anspruch 3, wobei das wärmeempfindliche gelbildende Polymer eine untere kritische Lösungstemperatur aufweist, die unter 30 °C, vorzugsweise unter 21 °C liegt.

5. Formulierung zur Verwendung nach einem der Ansprüche 1 bis 4, wobei das Polymer ein Poloxamer ist.

6. Formulierung zur Verwendung nach einem der Ansprüche 1 bis 5, wobei das Polymer in Kombination mit dem Hilfsstoff ein Gel auf einer Schleimhaut- oder Epitheloberfläche des Auges bildet.

7. Formulierung zur Verwendung nach einem der Ansprüche 1 bis 6, wobei der Wirkstoff wasserlöslich ist, oder wobei der Wirkstoff schwer wasserlöslich ist.

8. Formulierung zur Verwendung nach einem der Ansprüche 1 bis 6, wobei das Mittel hydrophob ist.

9. Formulierung zur Verwendung nach einem der Ansprüche 1 bis 8, wobei die hypotone Formulierung eine Tonizität von 50 mOsm/l bis 280 mOsm/l aufweist.

10. Formulierung zur Verwendung nach einem der Ansprüche 1 bis 9, umfassend Wasser und ein mit Wasser mischbares organisches Lösungsmittel, bei dem es sich um einen Alkohol, ein Keton, einen Ether oder einen Ester handeln kann, wobei optional:
(i) der Alkohol Ethanol oder Isopropanol ist;
(ii) das Keton Aceton ist;
(iii) der Ether Dioxan ist; oder
(iv) der Ester Ethylacetat ist.

11. Formulierung zur Verwendung nach einem der Ansprüche 1 bis 10, wobei die Formulierung das therapeutische, prophylaktische, diagnostische oder nutrazeutische Mittel an der epithelialen Oberfläche über einen Zeitraum von mindestens 12 Stunden oder über einen Zeitraum von mindestens 24 Stunden freisetzt.

12. Formulierung zur Verwendung nach einem der Ansprüche 1 bis 11, wobei das gelbildende Polymer zum Zeitpunkt der Verabreichung eine gleichmäßig dicke Schicht auf der epithelialen Oberfläche bildet.

13. Formulierung zur Verwendung nach einem der Ansprüche 1 bis 12 zur Verabreichung in Form eines Trockenpulvers, Gels oder einer Flüssigkeit, wobei die Formulierung optional in einer einzelnen oder mehreren Dosierungseinheiten zur Verabreichung bereitgestellt wird.

14. Formulierung zur Verwendung nach einem der Ansprüche 1 bis 13, wobei (i) der Wirkstoff ein Protein oder Peptid, ein kleines Molekül, ein Zucker oder Polysaccharid, ein Lipid, ein Glykolipid, ein Glykoprotein, eine Nukleinsäure, ein Oligomer oder Polymer davon oder ein kleines Molekül ist; oder
(ii) der Wirkstoff aus der Gruppe ausgewählt ist, die aus Steroiden, Glaukommitteln, Tyrosinkinase-Inhibitoren, Immunsuppressiva, antifibrotischen Mitteln, Antiinfektiva, Hormonen und Chemotherapeutika besteht.

15. Hypotonische Formulierung nach einem der Ansprüche 1 bis 14 zur Verwendung in der Medizin, wobei die Formulierung an einer Stelle verabreicht wird, die sie benötigt und die Stelle eine Schleimhaut- oder Epitheloberfläche des Auges ist.

16. Trockene Darreichungsform, umfassend ein schwer wasserlösliches therapeutisches, prophylaktisches, nutrazeutisches oder diagnostisches Mittel und ein gefriergetrocknetes Pulver, das zur Rekonstitution geeignet ist, um eine hypotonische Formulierung nach einem der Ansprüche 1 bis 14 zum Zeitpunkt der Anwendung zu bilden.

17. Hypotonische Formulierung zur Verwendung nach einem der Ansprüche 1 bis 14, hypotonische Formulierung zur Verwendung nach Anspruch 15 oder Trockendosierungsform nach Anspruch 16, wobei die Schleimhaut- oder Epitheloberfläche des Auges eines oder mehrere aus Hornhaut, Kammerwasser, Sklera, Bindehaut, Iris, Linse, Netzhaut und retinalem Pigmentepithel ist.

## Revendications

1. Formulation hypotonique pour une utilisation dans le traitement, la prévention ou le diagnostic d'une maladie oculaire comprenant l'administration à une surface muqueuse ou épithéliale de l'œil d'une formulation comprenant
un agent thérapeutique, prophylactique, nutraceutique ou diagnostique pour le traitement d'une maladie oculaire, un polymère gélifiant destiné à être appliqué sur l'œil, formulé de sorte que sa concentration soit inférieure à la concentration critique du gel (CCG) du polymère dans des conditions isotoniques et à une température comprise entre 25 et 37 °C au moment de l'administration, et que sa concentration augmente au fur et à mesure que l'eau est absorbée de la formulation jusqu'à ladite CCG ou au-dessus de celle-ci, formant ainsi un gel sur la surface muqueuse ou épithéliale, et
des excipients pour former une formulation hypotonique pharmaceutiquement acceptable du polymère convenant à l'administration dans l'œil d'un individu qui en a besoin,
dans lequel la formulation retient une concentration efficace dudit agent au niveau de la surface muqueuse ou épithéliale de l'œil,
dans laquelle le polymère gélifiant est (i) entre supérieur à 12 et inférieur à 24 % de poloxamère 288 (Pluronic^{®} F98) dans un excipient aqueux, ou (ii) est entre 10 et 18 % de poloxamère 407 (Pluronic^{®} F127).

2. Formulation pour utilisation selon la revendication 1 sous forme sèche ou liquide.

3. Formulation pour une utilisation selon l'une quelconque des revendications 1-2, dans laquelle le polymère gélifiant est un polymère gélifiant thermosensible.

4. Formulation pour une utilisation selon la revendication 3, dans laquelle le polymère gélifiant thermosensible a une température de solution critique inférieure à 30 °C, qui est de préférence inférieure à 21 °C.

5. Formulation pour une utilisation selon l'une quelconque des revendications 1-4, dans laquelle le polymère est un poloxamère.

6. Formulation pour une utilisation selon l'une quelconque des revendications 1-5, dans laquelle le polymère en combinaison avec l'excipient forme un gel sur une surface muqueuse ou épithéliale de l'œil.

7. Formulation pour une utilisation selon l'une quelconque des revendications 1-6, dans laquelle l'agent est soluble dans l'eau, ou dans laquelle l'agent est peu soluble dans l'eau.

8. Formulation pour une utilisation selon l'une quelconque des revendications 1-6, dans laquelle l'agent est hydrophobe.

9. Formulation pour une utilisation selon l'une quelconque des revendications 1-8, dans laquelle la formulation hypotonique a une tonicité de 50 mOsm/L à 280 mOsm/L.

10. Formulation pour une utilisation selon l'une quelconque des revendications 1-9, comprenant de l'eau et un solvant organique miscible à l'eau, qui peut être un alcool, une cétone, un éther ou un ester, éventuellement dans laquelle :
(i) l'alcool est l'éthanol ou l'isopropanol ;
(ii) la cétone est l'acétone ;
(iii) l'éther est du dioxane ; ou
(iv) l'ester est l'acétate d'éthyle.

11. Formulation pour une utilisation selon l'une quelconque des revendications 1-10, dans laquelle la formulation libère l'agent thérapeutique, prophylactique, diagnostique ou nutraceutique au niveau de la surface épithéliale sur une période d'au moins 12 heures, ou sur une période d'au moins 24 heures.

12. Formulation pour une utilisation selon l'une quelconque des revendications 1-11, dans laquelle le polymère gélifiant forme une couche uniformément épaisse au moment de l'administration sur la surface épithéliale.

13. Formulation pour une utilisation selon l'une quelconque des revendications 1-12 pour l'administration sous forme de poudre sèche, de gel ou de liquide, éventuellement dans laquelle la formulation est fournie dans une unité de dosage unique ou multiple pour l'administration.

14. Formulation pour une utilisation selon l'une quelconque des revendications 1-13 dans laquelle (i) l'agent est une protéine ou un peptide, une petite molécule, un sucre ou un polysaccharide, un lipide, un glycolipide, une glycoprotéine, un acide nucléique, un oligomère ou un polymère de ceux-ci, ou une petite molécule ; ou
(ii) l'agent est choisi dans le groupe constitué de stéroïdes, d'agents du glaucome, d'inhibiteurs de tyrosine kinase, d'agents immunosuppresseurs, d'agents antifibrotiques, d'anti-infectieux, d'hormones et d'agents chimiothérapeutiques.

15. Formulation hypotonique telle que définie dans l'une quelconque des revendications 1-14 pour une utilisation en médecine, dans laquelle la formulation est administrée à un site qui en a besoin et ledit site est une surface muqueuse ou épithéliale de l'œil.

16. Forme galénique sèche comprenant un agent thérapeutique, prophylactique, nutraceutique ou diagnostique peu soluble dans l'eau et une poudre lyophilisée pouvant être reconstituée pour former une formulation hypotonique telle que définie dans l'une quelconque des revendications 1-14 au moment de l'utilisation.

17. Formulation hypotonique pour une utilisation selon l'une quelconque des revendications 1-14, formulation hypotonique pour une utilisation selon la revendication 15, ou forme galénique sèche selon la revendication 16, dans laquelle la surface muqueuse ou épithéliale de l'œil est l'un ou plusieurs parmi la cornée, l'humeur aqueuse, la sclérotique, la conjonctive, l'iris, le cristallin, la rétine et l'épithélium pigmentaire rétinien.
